# FASCICULE DE BREVET EUROPEEN

(11) **EP 1 723 320 B1**
(45) Date de publication et mention de la délivrance du brevet: **28.07.2010**
(21) Numéro de dépôt: 05736932.4
(22) Date de dépôt: 11.03.2005
(51) Int. Cl.: F01M 11/10, G01F 1/704

(54) **PROCEDE ET DISPOSITIF DE MESURE EN TEMPS REEL A L'AIDE DE TRACEURS RADIOACTIFS DE LA CONSOMMATION D'HUILE DU SYSTEME DE SEPARATION D'HUILE MOTEUR**
VERFAHREN UND VORRICHTUNG ZUR ECHTZEITMESSUNG DES ÖLVERBRAUCHS VON EINEM MOTORÖLTRENNSYSTEM UNTER VERWENDUNG VON RADIOAKTIVEN TRACERN
METHOD AND DEVICE FOR THE REAL-TIME MEASUREMENT OF THE CONSUMPTION OF OIL FROM AN ENGINE OIL SEPARATION SYSTEM, USING RADIOACTIVE TRACERS

(30) Priorité: 11.03.2004 FR 0402541
(43) Date de publication de la demande: 22.11.2006
(73) Titulaire: TOTAL RAFFINAGE MARKETING, 92800 Puteaux (FR); Delta Services Industriels SPRL, 7503 Froyennes (BE)
(72) Inventeur: MARTIN, François, F-69003 Lyon (FR)
(74) Mandataire: Salou, Clarisse
(86) Numéro de dépôt international: PCT/FR2005/000590
(87) Numéro de publication internationale: WO 2005/088085

(56) Documents cités:
- DE-A- 19 631 654
- GB-A- 1 095 056
- US-A- 2 939 011
- US-A- 2 957 986
- BERGMANN M ET AL: "METHODE ZUR OELVERBRAUCHSMESSUNG DURCH MARKIERUNG MIT RADIOAKTIVEM BROM" MTZ MOTORTECHNISCHE ZEITSCHRIFT, FRANCKH'SCHE VERLAGSHANDLUNG,ABTEILUNG TECHNIK. STUTTGART, DE, vol. 58, no. 2, 1 février 1997 (1997-02-01), pages 102-107, XP000678384 ISSN: 0024-8525
- JOST K: "RADIOACTIVE MEASUREMENT TECHNIQUES FOR ENGINE TESTING" AUTOMOTIVE ENGINEERING, SOCIETY OF AUTOMOTIVE ENGINEERS. WARRENDALE, US, vol. 104, no. 3, 1 mars 1996 (1996-03-01), page 33, XP000583571 ISSN: 0098-2571

## Description

La présente invention concerne un procédé de mesure en temps réel de la consommation d'huile moteur liée au passage des gaz de carter à travers le système de séparation d'huile contenue dans lesdits gaz de carter.

Ce système de séparation d'huile est généralement constitué d'au moins un séparateur pouvant être notamment sous la forme d'un décanteur (séparateur) unique ou de plusieurs décanteurs (séparateurs) montés en série et/ou en parallèle, et constitue la pièce principale du circuit de recyclage des gaz de carter au sein des moteurs à combustion interne.

L'invention concerne en outre un dispositif, tel qu'un banc d'essai, permettant de mettre en oeuvre ce procédé. Plus précisément, ce procédé est basé sur la mesure en temps réel de la fraction d'huile moteur contenue dans les gaz de carter après leur passage au travers du système de séparation d'huile d'un moteur à combustion interne.

Les gaz de carter résultent en grande partie de la fuite des gaz de combustion hors du bloc moteur et, plus précisément, de la chambre de combustion, ces fuites étant dues aux jeux mécaniques dans la zone appelée communément « PSC » et constituée par le(s) piston(s), segment(s), cylindre(s) ou chemise (phénomène de *blow-by*)*.* Ces gaz contiennent des produits de combustion, de l'huile moteur, des hydrocarbures imbrûlés, des particules, de la vapeur d'eau etc.

Depuis de nombreuses années, sur les moteurs à combustion interne, les gaz de carter issus du bloc moteur, c'est à dire notamment du carter cylindres et/ou culasse, sont traités pour être ensuite soit libérés dans l'atmosphère (par exemple dans le cas des moteurs de poids lourds) soit recyclés vers l'admission et envoyés vers les cylindres pour y être brûlés (par exemple dans le cas des moteurs de véhicules légers).

Ce recyclage des gaz de carter vers le système d'admission comprend généralement :
(a) une étape de récupération de l'huile moteur contenue dans les gaz de carter, cette huile étant sous forme d'un mélange multiphasique liquide/vapeur dont les proportions sont variables en fonction des conditions de fonctionnement du moteur (ex: charge moteur, température, pression),
(b) une étape de renvoi de l'huile récupérée vers le carter d'huile moteur,
(c) puis une étape de renvoi des gaz de carter déshuilés vers l'admission ou de libération des gaz de carter déshuilés dans l'atmosphère.

La séparation de l'huile d'avec les gaz de carter dans l'étape (a) se fait dans un système de séparation usuellement appelé "décanteur " ou "décanteur moteur". A cet effet, de nombreuses variantes technologiques de systèmes de séparation ont été développées. Par exemples, l'huile peut être retenue par une surface de contact importante fournie par des éléments statiques tels que des chicanes ou des éléments de garnissage, des éléments filtrants, ou l'huile peut être retenue grâce à la force centrifuge d'un ou de plusieurs cyclones projetant les gouttelettes contre la surface du séparateur.

Le document DE 196 31654 décrit un décanteur faisant partie du circuit de désaération de l'huile d'un carter de moteur. Ce décanteur sépare l'huile d'un mélange gaz/huile provenant du carter de moteur lorsque la pression à l'intérieur de ce dernier est trop élevée, et la renvoie, après filtration, au carter moteur. Le gaz séparé au niveau du décanteur est quant à lui rejeté dans l'atmosphère.

Toutefois, l'efficacité de tels séparateurs n'est généralement pas totale, c'est-à-dire une fraction plus ou moins importante de l'huile contenue dans les gaz de carter n'est pas retenue au sein du système de séparation. De ce fait, le renvoi à l'admission des gaz de carter contenant encore de l'huile après passage dans le système de séparation entraîne un certain nombre de nuisances techniques susceptibles de gêner le bon fonctionnement du moteur à combustion interne.

Ainsi, les risques d'encrassement du moteur sont sensiblement augmentés. On peut observer par exemple, l'encrassement des tubulures d'admission, papillon(s), vanne(s) EGR, soupapes et chambres de combustion. Les risques d'effets mécaniques gênants tels que grippage du ou des papillon(s), grippage de la ou des vanne(s) EGR, cliquetis, etc. sont de ce fait augmentés.

En outre, l'encrassement moteur est susceptible d'augmenter les émissions polluantes (ex. : combustion incomplète de l'huile avec d'éventuelles conséquences sur les systèmes de post-traitement) et d'avoir un impact non négligeable sur l'agrément de conduite du véhicule motorisé.

Par conséquent, les constructeurs automobiles dans un souci de développer de nouveaux systèmes de séparation d'huile comprenant un ou plusieurs séparateurs en série et/ou en parallèle doivent être en mesure de contrôler aisément et efficacement les performances de ceux-ci. Un tel contrôle exige un procédé de mesure de la consommation d'huile dudit système de séparation qui doit être fiable, précis, répétable et reproductible.

Pour évaluer cette consommation d'huile au sein du système de séparation, il s'agit de mesurer la fraction d'huile encore présente dans les gaz de carter après le passage desdits gaz dans ce système de séparation. Plus le système de séparation sera efficace, plus la fraction d'huile mesurée sera faible.

A cet effet, la Demanderesse a mis au point un nouveau procédé de mesure de la consommation d'huile issue du système de séparation d'huile situé dans le circuit de recyclage des gaz de carter d'un moteur à combustion interne, lequel procédé est de mise en oeuvre aisée, automatisable, fiable, précis, répétable, reproductible et peut fonctionner en continu de manière à fournir quasiment instantanément et en temps réel la fraction d'huile non récupérée par le système de séparation.

Le procédé selon l'invention facilite ainsi la réalisation d'essais en vue du développement de nouveaux systèmes de séparation permettant un déshuilage optimal des gaz de carter.

Le procédé d'analyse de la présente invention est basé sur l'introduction d'un traceur radioactif dans l'huile de lubrification du moteur et sur la mesure de la radioactivité de l'huile contenue dans les gaz de carter sortant du système de séparation constitué d'un ou de plusieurs séparateurs d'huile dont on souhaite évaluer l'efficacité.

Le document US2 957,986 décrit un procédé de détermination de la consommation d'huile d'un moteur à combustion interne, comprenant le marquage radioactif de l'huile moteur par du ¹⁴C et la mesure de la radioactivité rejetée par les gaz d'échappement ou de la radioactivité contenue dans l'huile du carter de moteur. Ce document ne prévoit pas l'utilisation du marquage radioactif pour déterminer la consommation d'huile d'un système de séparation d'huile faisant partie d'un circuit de recyclage des gaz de carter.

Sous réserve de respecter certaines conditions concernant le choix du radiotraceur, cette radioactivité reflète en effet parfaitement la fraction d'huile de lubrification encore contenue dans les gaz de carter après leur passage dans le système de séparation. La mesure de cette radioactivité permet ainsi de déterminer en temps réel l'efficacité du système de séparation.

La présente invention a donc pour objet un procédé de détermination de la consommation d'huile issue du système de séparation d'huile, situé dans le circuit de recyclage des gaz de carter d'un moteur à combustion interne, tel que défini par la revendication 1.

La consommation d'huile peut être exprimée sous différentes formes selon le type de tests effectués. Elle peut être notamment exprimée :
- en masse (grammes),
- en volume (litres),
- en masse par unité de temps (débit massique: par exemple grammes/heure),
- en volume par unité de temps (débit volumique: par exemple litres/heure),
- en masse par unité de distance parcourue par le véhicule (par exemple grammes/km),
- ou encore en volume par unité de distance parcourue par le véhicule (ex: litres/km)

L'invention a en outre pour objet un dispositif selon la revendication 12.

Le procédé et le dispositif de la présente invention apportent une grande flexibilité et précision de mesure, et permettent, grâce à leur grande sensibilité, de mesurer rapidement et quasi instantanément la consommation d'huile par le système de séparation des gaz de carter. Ils présentent en outre l'avantage de ne pas perturber le fonctionnement du moteur testé et permettent ainsi une bonne extrapolation des résultats d'essais aux conditions de fonctionnement réel du moteur.

Dans un premier mode de réalisation de la présente invention, on mesure directement, à l'aide d'un détecteur situé à proximité de la conduite de sortie du système de séparation d'huile à tester, la fraction d'huile radioactive résiduelle contenue dans les gaz de carter partiellement déshuilés suite à leur passage dans le système de séparation.

Ce mode de réalisation, mesurant de faibles quantités de radioactivité, peut impliquer l'utilisation de radiotraceurs ayant une activité spécifique importante ou bien l'utilisation de concentrations importantes de radiotraceurs à activité moyenne. Dans un tel cas, il est bien entendu indispensable ne prendre des mesures de protection appropriées, permettant de limiter l'impact de la radioactivité sur l'environnement.

Si le détecteur utilisé a toutefois une sensibilité élevée, l'activité spécifique ou la quantité des radiotraceurs utilisés ne doit pas forcément être très élevée.

Cette méthode de mesure présente l'avantage d'une perturbation nulle du fonctionnement du moteur car elle ne modifie aucunement l'écoulement fluidique dans le circuit de recyclage des gaz de carter.

Un deuxième mode de réalisation de la présente invention consiste à mesurer l'accroissement de la radioactivité due à l'accumulation de l'huile radioactive dans un dispositif de piégeage approprié. Concrètement, dans ce deuxième mode de réalisation, on piège l'huile résiduelle des gaz de carter partiellement déshuilés sortant du système de séparation d'huile dans un dispositif de piégeage d'huile situé en aval dudit système de séparation d'huile et l'on mesure la radioactivité globale de l'huile ainsi recueillie.

L'ajout d'un dispositif de piégeage d'huile dans le circuit de recyclage des gaz de carter est susceptible de modifier l'écoulement fluidique dans celui-ci. Une bonne extrapolation des résultats d'essais aux conditions de fonctionnement réel implique par conséquent de réduire au minimum une telle perturbation potentielle en choisissant une géométrie appropriée pour ce dispositif de piégeage (faible volume, longueur minimale des conduites etc.). Le paramètre reflétant le mieux la perturbation de l'écoulement fluidique dans le circuit de recyclage des gaz de carter est la différence de pression des gaz de carter (ΔP) entre l'entrée et la sortie du système de séparation d'huile. Lorsque la valeur de ce paramètre est sensiblement identique à la différence de pression en l'absence du dispositif de piégeage, la perturbation de l'écoulement fluidique par le dispositif est nulle, ce qui correspond à un mode de réalisation préféré.

Ce deuxième mode de réalisation utilisant un dispositif de piégeage présente l'avantage considérable de permettre des mesures d'une grande précision avec des faibles concentrations de radiotraceurs ou avec des radiotraceurs relativement peu actifs, qui présentent de ce fait une mise en oeuvre facile sans risque pour l'environnement et le personnel chargé des essais.

Le dispositif de piégeage d'huile mentionné ci-dessus est de préférence constituée d'au moins un séparateur fonctionnant selon le même principe que le système de séparation d'huile à tester. Le dispositif de piégeage d'huile de la présente invention comporte par exemple un ou plusieurs éléments statiques de séparation, tels que des chicanes ou des éléments de garnissage, fournissant une grande interface solide/gaz, et/ou des éléments filtrants et/ou un ou plusieurs cyclones permettant grâce à la force centrifuge de capter l'huile.

Dans certaines conditions de fonctionnement du moteur, il peut être utile de refroidir le dispositif de piégeage d'huile au moyen d'un système de réfrigération approprié afin d'améliorer l'efficacité de piégeage de ce dispositif.

En outre, on comprendra que plus l'efficacité du dispositif de piégeage est élevée et tend vers 100 %, plus la mesure de radioactivité de l'huile accumulée dans le dispositif de piégeage reflète correctement la consommation d'huile par le système de séparation moteur. Il existe notamment des dispositifs de piégeage retenant la majorité, c'est-à-dire au moins 80 % ou idéalement au moins 95 %, de la fraction d'huile contenue dans les gaz de carter sortant du système de séparation moteur. On peut toutefois également mesurer de manière fiable et reproductible la consommation d'huile liée au passage des gaz de carter au travers du système de séparation d'huile avec des dispositifs de piégeage ayant une efficacité moindre établie préalablement par étalonnage, à condition notamment de connaître le rapport de la quantité d'huile piégée par rapport à la quantité d'huile entrant et que ce rapport soit connu en fonction des différentes conditions expérimentales utilisées.

Le procédé de mesure de la consommation d'huile moteur liée au passage des gaz de carter au travers du système de séparation d'huile d'un moteur à combustion interne doit être mis en oeuvre avec un traceur radioactif approprié. Celui-ci doit remplir notamment les conditions énoncées dans les paragraphes ci-après.

Le radiotraceur ne doit pas perturber le fonctionnement du moteur ou modifier de manière gênante les propriétés physico-chimiques de l'huile moteur. Pour cela il doit notamment soit être chimiquement inerte vis-à-vis des composants de ceux-ci, soit avoir une fonction similaire à celle d'un de leurs constituants (par exemple un additif fonctionnel) et se substituer partiellement ou totalement à celui-ci.

Le radiotraceur doit avoir une radioactivité suffisante pour permettre des mesures précises et reproductibles. Le choix du radiotraceur est lié notamment à la sensibilité du détecteur utilisé. En d'autres termes, si le détecteur est peu sensible, la radioactivité de l'huile doit être élevée (radioactivité forte du radiotraceur ou concentration élevée d'un radiotraceur de radioactivité relativement faible). Par contre, si le détecteur utilisé a une sensibilité élevée, la radioactivité de l'huile peut être relativement plus faible.

Enfin, le radiotraceur doit être sélectionné de manière à ce que sa quantité en circulation dans le circuit d'huile du moteur soit, sur toute la durée du procédé, directement proportionnelle à la quantité d'huile de lubrification dans le circuit d'huile du moteur.

Cette proportionnalité dépend des propriétés physico-chimiques du traceur radioactif et de celles du milieu liquide dans lequel il est introduit initialement. En effet, pour refléter à tout moment la fraction d'huile moteur mesurée, le traceur radioactif ne doit ni s'accumuler dans le mélange lorsque l'huile est consommée, ni se consommer plus rapidement que celle-ci, par exemple par évaporation, combustion ou décomposition thermique, ni être piégé en un endroit quelconque du moteur, tel que le filtre à huile.

A la lumière de ce qui précède, l'homme du métier choisira le radiotraceur tel que ses propriétés physico-chimiques (volatilité, stabilité thermique, réactivité chimique) soient en adéquation avec celles du milieu liquide dans lequel il est introduit et dont il doit refléter la fraction mesurée.

L'homme du métier pourra notamment trouver un traceur approprié pour un milieu donné en soumettant un mélange traceur/huile lubrifiante aux conditions de température et de pression qui règnent dans un moteur.

Les détecteurs utilisables sont des sondes de détection de rayonnements ionisants (rayons bêta, X ou gamma) pouvant être soit de type scintillateur liquide ou solide (cristal iodure de sodium NaI(Tl), cristal BGO), soit de type semi-conducteur (cristal germanium, cristal CZT). On notera en outre que le détecteur peut déceler simultanément la présence de divers radiotraceurs. Lorsque la radioactivité de l'huile contenue dans les gaz de carter est élevée (radioactivité forte du radiotraceur et/ou concentration élevée d'un radiotraceur de radioactivité faible), le détecteur ne nécessitera pas une sensibilité élevée. Par contre, lorsque la radioactivité de l'huile contenue dans les gaz de carter n'est pas élevée, le détecteur nécessitera une sensibilité plus élevée.

Afin de limiter la quantité de radiotraceurs mis en oeuvre, on utilisera de préférence une sonde de mesure dont l'efficacité de détection est élevée, par exemple un cristal de type iodure de sodium de 3 x 3 pouces.

Ce type de détecteur peut exister sous forme compacte permettant la possibilité d'un dispositif embarqué sur véhicule.

Les signaux détectés par le détecteur sont ensuite traités par une série de moyens permettant de calculer la fraction d'huile moteur contenue dans les gaz de carter après leur passage au travers du système de séparation d'huile. Ces moyens comprennent notamment un moyen de traitement du signal détecté (par exemple un amplificateur, un filtre et un convertisseur analogique/numérique CAD), un moyen de traitement d'impulsions (par exemple un analyseur multi-canaux) et un moyen de stockage et de traitement des données acquises (par exemple un ordinateur PC).

La mesure de la radioactivité et le traitement des résultats se font de préférence en continu.

Le traceur radioactif utilisable dans la présente invention peut être soit un composé organique ou minéral d'un élément radioactif (radionucléide) soit l'élément radioactif lui-même qui est alors sous forme élémentaire. Toutefois, compte tenu des considérations ci-dessus concernant les propriétés physico-chimiques du traceur radioactif par rapport à celles de l'huile de lubrification, les formes moléculaires, organiques ou minérales, de radiotraceurs sont préférées par rapport aux formes élémentaires des radionucléides.

Le radiotraceur est donc choisi parmi les composés organiques ou minéraux ou les éléments remplissant les conditions indiquées précédemment (i.e. caractère inerte vis-à-vis du lubrifiant ou substitution à l'un des composants du lubrifiant, radioactivité suffisante et proportionnalité huile/traceur). Toutefois, pour des raisons évidentes de manipulation et de protection de l'environnement, on choisira de préférence des traceurs contenant des radionucléides ayant une courte période, ou demi-vie, de préférence une période inférieure à 3 ans, en particulier inférieure à 1 an et encore plus préférentiellement inférieure à 30 jours. De cette manière, on évitera la production de déchets radioactifs à longue demi-vie.

Il est préférable que la période du radionucléide soit égale ou supérieure à la durée prévue de l'essai. L'ordinateur, grâce à là loi de décroissance radioactive pourra corriger facilement la valeur mesurée.

On peut citer à titre d'exemples de radionucléides ayant une période (indiquée entre parenthèses) appropriée : le ²²Na (2,61 ans), le ⁶⁵Zn (243,8 jours), le ⁴⁵Ca (165 jours), le ³⁵S (87,2 jours), le ³²P (14,3 jours), le ⁴⁷Ca (4,54 jours), le ⁹⁹Mo (65,9 heures), le ⁸²Br (35,3 heures), le ⁶⁴Cu (12,7 heures), le ^{99m}Tc (6,01 heures), le ²⁸Mg (20,91 heures), le ⁶⁸Ge (270,95 jours), le ⁶⁹Ge (39h), le ⁷⁷Ge (11,30 heures), le ⁸⁵Sr (64,8 jours) et le ⁵⁶Co (77,3 jours).

Ces radiotraceurs sont en général produits artificiellement par réactions nucléaires, notamment par réaction d'activation. Cette activation se fait selon des méthodes familières à l'homme du métier, par exemple par exposition des éléments inactifs ou composés contenant lesdits éléments inactifs à une source de rayonnement neutronique, ou encore par exposition à un faisceau d'ions accélérés provenant d'un accélérateur de particules.

Selon les cas, les éléments inactifs ou composés contenant lesdits éléments inactifs sont activés soit avant leur incorporation dans l'huile moteur, soit au sein même de l'huile, c'est-à-dire en exposant l'huile contenant l'élément ou le composé activable par exemple à un rayonnement neutronique ou à un faisceau de protons.

Une des options possibles pour obtenir des radionucléides artificiels est d'incorporer les éléments inactifs ou composés contenant lesdits éléments inactifs dans une quantité appropriée d'un vecteur (par exemple un solvant ou diluant tel qu'une huile), puis de soumettre ce mélange à l'activation et enfin de l'ajouter à l'huile lubrifiante.

Les radiotraceurs peuvent être des additifs utilisés habituellement dans les huiles de lubrification, tels que des agents anti-corrosion, des agents anti-oxydants, des agents modifiant la viscosité, des additifs lubrifiants, des colorants, des additifs abaissant le point d'écoulement, des additifs détergents ou dispersants. On peut citer à titre d'exemples de tels radiotraceurs fonctionnant comme un additif fonctionnel, le dithiophosphate de zinc, les sulfonates de calcium ou de magnésium, tels que les alkylsulfonates, arylsulfonates ou alkylarylsulfonates de calcium ou de magnésium, les phénates de calcium, les phénates de magnésium, les salicylates de calcium, les salicylates de magnésium.

Cependant, l'utilisation de radiotraceurs qui n'ont aucune fonction physique ou chimique dans le système de lubrification du moteur est tout aussi appropriée.

La Demanderesse a constaté que les traceurs radioactifs particulièrement intéressants pour une introduction dans l'huile moteur sont certains composés du germanium-69. Ces composés sont choisis par exemple parmi les tétra-alkylgermanes. Le point d'ébullition de ces tétra-alkylgermanes étant proportionnel à la longueur des chaînes alkyle, on utilisera avantageusement un mélange de tétraalkylgermanes ayant des chaînes alkyle telles que le point d'ébullition du mélange est situé dans l'intervalle de distillation de l'huile utilisée. A titre d'exemples, le tétrahexylgermane, le tétraheptylgermane et le tétraoctylgermane ont chacun un point d'ébullition comparable à celui d'un lubrifiant moteur classique.

Un autre radiotraceur particulièrement intéressant en particulier en raison de sa facilité de préparation et de manipulation, et de sa très courte durée de vie est le ^{99m}Tc, de préférence sous forme d'une solution aqueuse de pertechnétate de sodium NaTcO₄ ou sous forme de particules de dimensions nanométriques et isolées de l'atmosphère par du carbone.

L'invention est maintenant décrite en référence aux dessins annexés, non limitatifs, dans lesquels :
La figure 1 représente une première variante du procédé de mesure de la consommation d'huile selon l'invention dans lequel l'huile radioactive contenue dans les gaz de carter sortant du système de séparation d'huile est piégée dans un dispositif de piégeage ;
La figure 2 représente une deuxième variante du procédé de mesure de la consommation d'huile selon l'invention dans lequel on mesure directement la radioactivité de l'huile radioactive contenue dans les gaz de carter sortant du système de séparation d'huile sans piégeage préalable de l'huile ;
La figure 3 représente une troisième variante du procédé de mesure de la consommation d'huile selon l'invention qui diffère du procédé de la figure 1 par le fait que les gaz de carter traités ne sont pas renvoyés vers l'admission mais libérés dans l'atmosphère.
La figure 4 est un graphique représentant la quantité d'huile piégée dans un premier dispositif de piégeage, disposé en aval d'un système de séparation d'huile d'un circuit de recyclage des gaz de carter d'un moteur à combustion interne, en fonction du nombre de kilomètres parcourus par le véhicule.
La figure 5 est un graphique représentant la quantité d'huile piégée dans un deuxième dispositif de piégeage, disposé en aval d'un système de séparation d'huile d'un circuit de recyclage des gaz de carter d'un moteur à combustion interne, en fonction du nombre de kilomètres parcourus par le véhicule.

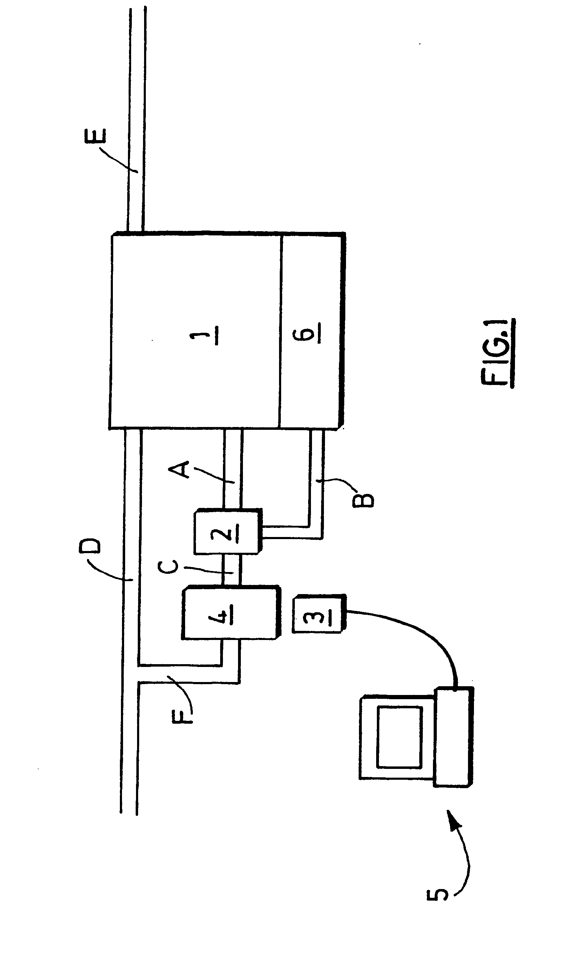

Sur l'ensemble des schémas des figures 1 à 3, le système de séparation d'huile est illustré sous la forme d'un séparateur unique. Dans le cas où le système de séparation comprend deux séparateurs ou plus montés en série, une mesure directe peut être effectuée à la sortie de chaque séparateur. Alternativement, une mesure unique peut être effectuée à la sortie du séparateur le plus en aval, avec ou sans piégeage de l'huile par un dispositif de piégeage, pour calculer la consommation globale du système.

Le moteur à combustion interne peut aussi comporter plusieurs systèmes de séparation montés en parallèle (par exemple moteur V6). Dans ce cas, la mesure de la consommation peut être faite soit sur l'ensemble des gaz de carter réunis en aval des systèmes de séparation, avec ou sans piégeage de l'huile, soit en sortie de chaque système de séparation (pouvant chacun comprendre également plusieurs séparateurs) lorsque les gaz de carter issus de chaque système de séparation ne sont pas réunis dans une conduite unique mais sont renvoyés séparément vers l'admission ou dans l'atmosphère.

L'invention est décrite maintenant en référence à la figure 1. Dans ce mode de réalisation, les gaz de carter chargés d'huile marquée par au moins un traceur radioactif sortent du bloc moteur 1 et parviennent, via la conduite d'entrée A, dans le séparateur d'huile 2. Dans ce séparateur d'huile 2 s'effectue la séparation de l'huile contenue dans ces gaz de carter. L'huile ainsi séparée est renvoyée vers le carter d'huile 6 via la conduite de retour B.

Les gaz de carter imparfaitement déshuilés et contenant encore une certaine fraction d'huile radioactive sont envoyés via la conduite de sortie C vers le dispositif de piégeage d'huile 4 où l'huile résiduelle est retenue. Les gaz de carter à présent totalement ou presque totalement déshuilés sont renvoyés via la conduite F vers l'admission D.

Un détecteur 3, sensible au rayonnement émis par le ou les radiotraceur(s) introduit(s) dans l'huile de lubrification, est placé à proximité immédiate du dispositif de piégeage 4 contenant l'huile séparée. Ce détecteur doit être protégé par un matériau approprié, par exemple du plomb, contre le rayonnement émis par l'huile de lubrification radioactive en circulation dans le moteur situé à proximité. Ainsi protégé contre ce rayonnement parasite, le détecteur 3 mesure uniquement la radioactivité qui s'accumule dans le dispositif de piégeage 4.

Le détecteur 3 est relié à un système informatique 5 d'enregistrement et de traitement des données de mesure, programmé pour calculer la consommation d'huile engendrée par le système de séparation d'huile 2 dans le système de recyclage des gaz de carter.

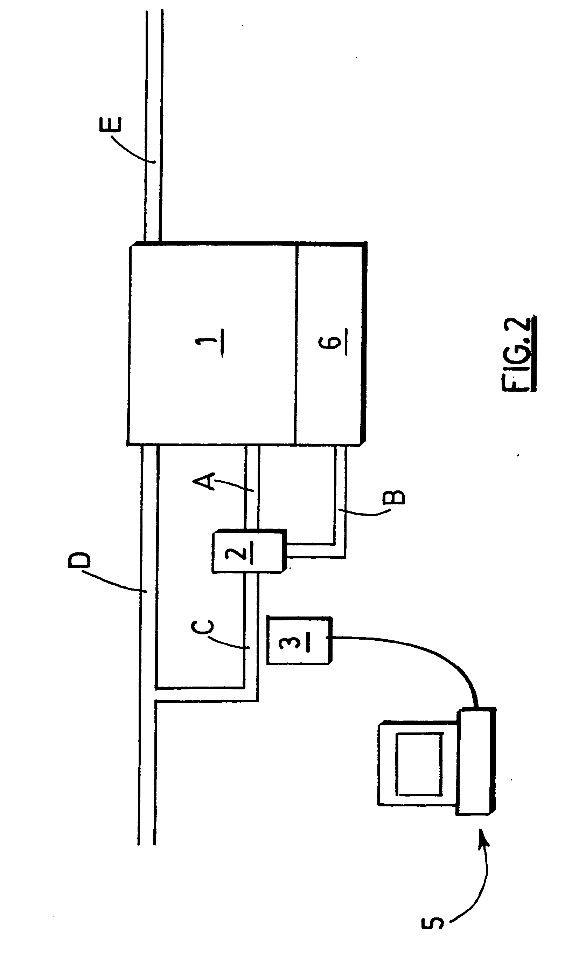

Le procédé de la figure 2 diffère de celui représenté à la figure 1 par le fait que les gaz de carter sortant via la conduite de sortie C du système de séparation d'huile 2 sont directement recyclés vers l'admission. La mesure de la radioactivité par le détecteur 3 relié au système informatique 5 se fait sans piégeage préalable de l'huile résiduelle présente dans les gaz de carter en sortie du système de séparation 2. Le détecteur 3 est placé à proximité de la conduite C où il mesure la radioactivité du flux de gaz de carter dans cette conduite. Ce mode de réalisation, contrairement à celui de la figure 1, est parfaitement non-intrusif, c'est-à-dire n'implique aucune modification de l'écoulement des gaz dans le moteur et dans le système de recyclage des gaz de carter.

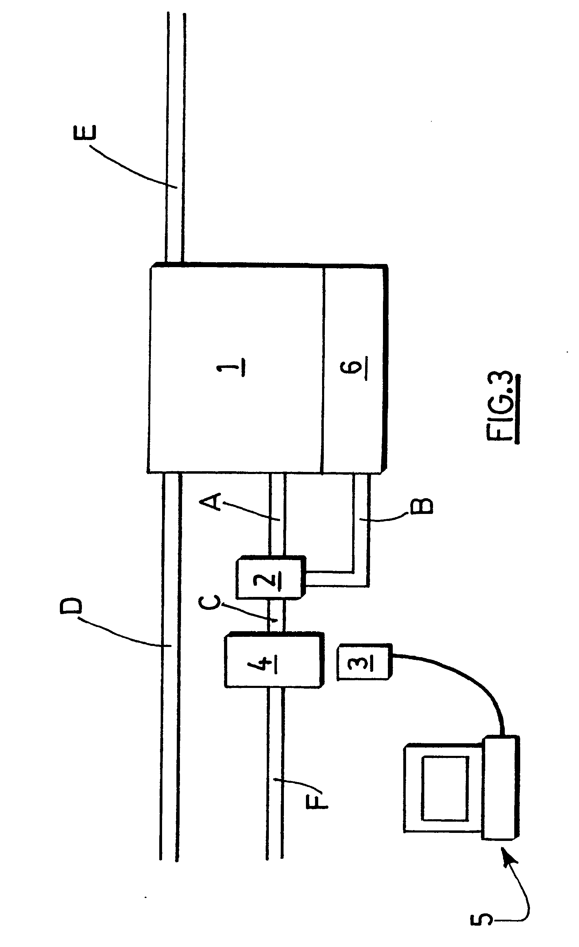

Enfin, le procédé représenté à la figure 3 est sensiblement identique à celui de la figure 1, à ceci près que les gaz de carter traités (déhuilés) ne sont pas recyclés vers l'admission D mais libérés dans l'atmosphère.

L'invention sera encore mieux comprise à la lecture des exemples suivants qui ont un caractère purement illustratif et ne limitent aucunement la portée de la présente invention.

### Exemples

Ces exemples constituent des configurations d'essai. Ils sont destinés à illustrer le procédé de mesure en temps réel de la consommation d'huile moteur lié au passage des gaz de carter à travers le système de séparation de l'huile contenue dans lesdits gaz.

Le moteur utilisé dans ces essais est un moteur fonctionnant à l'essence, 4 cylindres/8 soupapes de type « F3R » installé sur un véhicule de type Renault Espace, d'une cylindrée de 2 litres.

L'huile pour moteur à quatre temps est une huile commercialisée sous la marque ELF, type Evolution SXR 5W30.

On a utilisé comme traceur radioactif le Germanium-69, préparé sous la forme d'un complexe de tétraalkylgermanes solubles dans l'huile dont l'intervalle de distillation est représentatif du lubrifiant. L'activité totale mise en oeuvre est de 37 MBq (1 mCi), le traceur étant mélangé aux 5 litres d'huile du carter moteur.

Le piège à huile utilisé est installé près du moteur, entre la sortie du décanteur d'huile et le retour vers le collecteur d'admission.

Deux types de pièges ont été utilisés correspondant aux 2 exemples ci-dessous. Pour l'exemple 1, la cartouche utilisée contient une laine animale haute densité imprégnée de résine (laine filtrante commercialisée par la société Vita Medical Ltd, Australie). Le piège utilisé pour l'exemple 2 est constitué de limailles d'inox (fines spirales enchevêtrées). Le volume total de chaque type de piège est de l'ordre de 100 ml.

Les essais ont été effectués dans des conditions identiques et répétables (3 km sur route + 7 km sur autoroute, changements de rapports à des régimes moteurs voisins, même vitesse moyenne sur autoroute, etc.).

Après chaque trajet, le véhicule est rentré dans l'atelier et une sonde de mesure de radioactivité a été repositionnée de façon identique à proximité du piège afin d'évaluer la fraction d'huile accumulée dans le filtre via une mesure de rayonnement gamma (raie située à 511 keV).

Des précautions ont été prises afin de focaliser la sonde de mesure sur le rayonnement émis par le piège, tout en minimisant l'impact du signal en provenance de l'huile contenue dans le moteur. Un épais blindage de plomb a été utilisé à cet effet et la sonde a été positionnée contre le piège, mais le plus loin possible du bloc moteur.

Plusieurs points de mesure ont été effectués après chaque trajet, le temps de comptage étant de 30 secondes par point de mesure.

Le système de détection du rayonnement est constitué d'un détecteur standard NaI(Tl) de 3*3 pouces avec tube photomultiplicateur intégré, les autres éléments de la chaîne de mesure étant un préamplificateur de charge modèle 2007P de marque Canberra, un amplificateur de spectroscopie 2020 (Canberra), un convertisseur ADC modèle 8087 (Canberra) et une carte multicanaux modèle S 100 (Canberra).

Les logiciels mis en oeuvre lors de ces essais sont « Génie 2000 » (Canberra) pour la spectrométrie gamma, ainsi que le logiciel d'analyse MCS (Multi Channel Scaling) « IDSWear » commercialisé par la société Atlantic Nuclear Services (ANS), Canada. Les résultats présentés ci-dessous incluent une correction en demi-vie (qui est 39 heures pour le Ge-69) par le logiciel de mesure « IDSWear ».

Il faut noter que la procédure ici appliquée est de type quasi continue, c'est-à-dire chaque point de mesure a été réalisé à intervalle de temps et de distance régulier et identique.

### Exemple 1

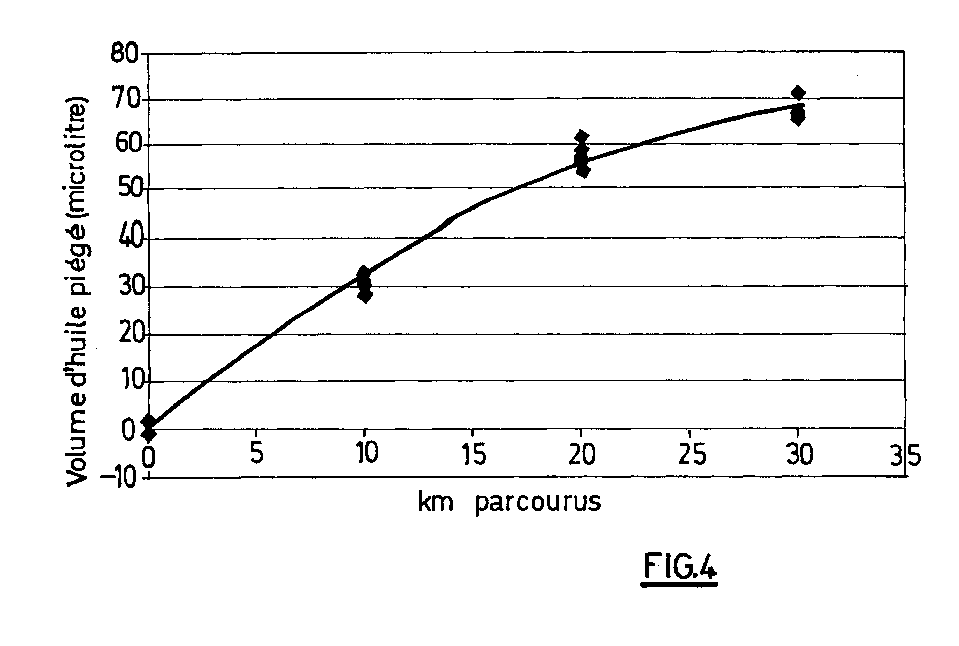

Le diagramme de la figure 4 montre la quantité d'huile piégée dans un dispositif de piégeage situé en aval d'un système de séparation d'huile, en fonction du nombre de km parcourus par le véhicule.

Le volume d'huile piégé a été calculé sur base de l'activité spécifique de l'huile du moteur (qui était de 7.4 Bq par microlitre en début d'essai), en tenant compte de l'efficacité du détecteur et de la géométrie de détection.

L'efficacité globale sur le pic de 511 keV (environ 7%) a été évaluée via un code de calcul de type "Monte Carlo" dont les paramètres d'entrée ont été adaptés à la configuration de mesure.

L'augmentation de l'activité détectée en fonction du temps est ainsi directement convertie en volume d'huile piégé. On constate sur le diagramme que le volume piégé ne croît pas linéairement en fonction du nombre de km parcourus.

Ce résultat s'explique par le fait que la laine imprégnée contenue dans le piège offre également une bonne efficacité de piégeage pour les vapeurs d'eau. De ce fait, la laine s'est peu à peu imprégnée d'eau, ce qui a eu pour conséquence une circulation des gaz de plus en plus difficile dans le piège, et donc un volume d'huile piégé par km parcouru qui décroît avec le temps.

On peut néanmoins relever, en début d'essai, une consommation d'huile voisine de 3 microlitres par km parcouru.

### Exemple 2

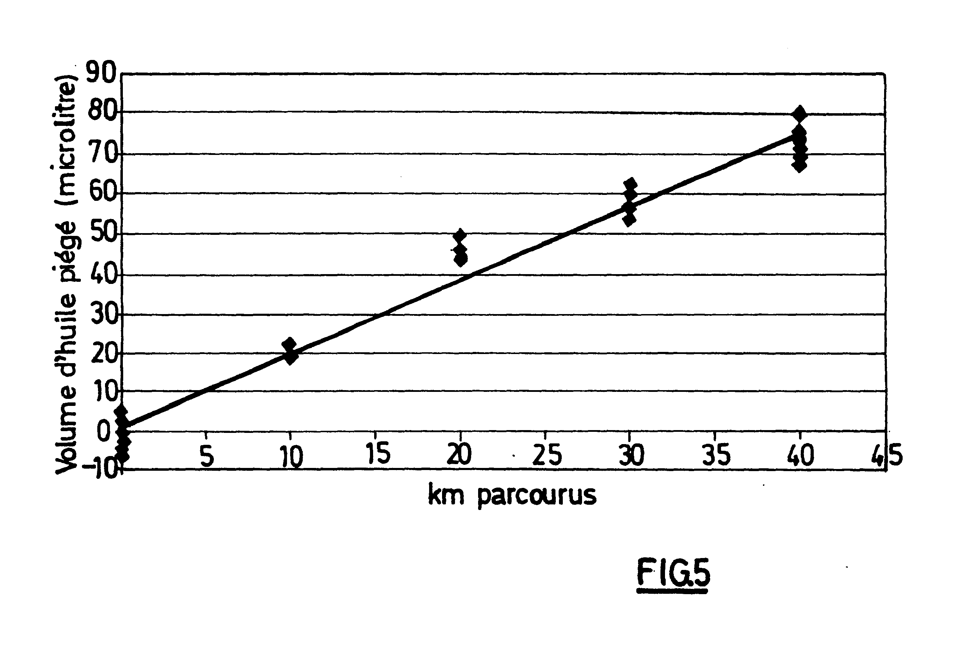

La figure 5 est un diagramme montrant la quantité d'huile piégée dans un dispositif de piégeage contenant de la limaille d'inox, situé en aval d'un système de séparation d'huile, en fonction du nombre de km parcourus.

Le volume d'huile piégé a été calculé sur base de l'activité spécifique de l'huile du moteur (qui était de 5.48 Bq par microlitre en début d'essai), en tenant compte de l'efficacité du détecteur et de la géométrie de détection.

L'efficacité globale sur le pic de 511 keV (environ 7%) a été évaluée via un code de calcul de type « Monte Carlo » dont les paramètres d'entrée ont été adaptés à la configuration de mesure.

L'augmentation d'activité détectée en fonction du temps est ainsi directement convertie en volume d'huile piégé, et l'on constate sur le diagramme que le volume piégé croît quasi linéairement en fonction du nombre de km parcourus.

Les écarts par rapport à la droite de régression constatés pour la troisième série de mesures effectuées à 20 km s'expliquent par des conditions de circulation qui ont légèrement varié.

La bonne linéarité de la courbe obtenue s'explique par le fait que le matériau utilisé n'a ici aucune tendance à accumuler l'eau contenue dans les gaz de carter. Dès lors, la résistance du piège à la circulation des gaz ne varie pas dans le temps.

La consommation d'huile moyenne relevée est de 1,875 microlitre par km parcouru, valeur inférieure à celle relevée dans l'exemple 1. Cela s'explique par un maillage nettement plus grand pour le filtre inox qui ne permet pas de piéger la totalité de l'huile résiduelle sortant du système de séparation d'huile.

### Exemple 3

Cet exemple est destiné à illustrer le procédé de mesure en temps réel de la consommation d'huile moteur lié au passage des gaz de carter (gaz de *blow-by*) à travers le système de séparation de l'huile contenue dans lesdits gaz.

Le moteur utilisé dans cet essai est un moteur fonctionnant à l'essence, 6 cylindres/24 soupapes de type « L7X » installé sur un banc d'essai moteur, d'une cylindrée de 3 litres.

L'huile pour moteur à quatre temps est une huile commercialisée sous la marque TOTAL, type LUB MA3 5W-30.

Le traceur radioactif utilisé est le Technétium ⁹⁹Tc (rayonnement gamma à 141keV), préparé sous la forme d'un mélange d'huile décrite ci-dessus et de nanoparticules de carbones enfermant du ⁹⁹Tc. L'activité spécifique du mélange d'huile/⁹⁹Tc utilisé dans cet essai est de 2 kBq/ml (2.3 kBq/g) soit une activité totale dans le moteur d'environ 14.000 kBq.

Le piège à huile utilisé est installé près du moteur, entre la sortie du décanteur d'huile et le retour vers le collecteur d'admission. Ce piège est de type cyclone d'un volume de l'ordre de 10 mL occasionnant une perte de charge inférieure à 10mbar, qui est compensée par l'utilisation de tubes de liaison sans perte de charge afin de garantir un écoulement identique au système moteur standard. L'huile piégée à partir des effluents est captée dans un flacon d'une contenance de 100 mL.

La mesure a été réalisée en temps réel et en continu, l'ordinateur étant programmé pour calculer une valeur moyenne par période de mesure de 5 minutes. La chaîne de détection est restée dans la même configuration durant tout l'essai.

Des précautions ont été prises afin de focaliser la sonde de mesure sur le rayonnement émis par le piège, tout en minimisant l'impact du signal en provenance de l'huile contenue dans le moteur. Un épais blindage de plomb a été utilisé à cet effet et la sonde a été positionnée contre le piège et le plus loin possible du bloc moteur.

Le système de détection du rayonnement est constitué d'un détecteur standard NaI(Tl) de 2x2 pouces avec tube photomultiplicateur intégré, les autres éléments de la chaîne de mesure étant un préamplificateur de charge modèle 2007P de marque Canberra, un amplificateur de spectroscopie 2020 (Canberra), un convertisseur ADC modèle 8087 (Canberra) et une carte multicanaux modèle S 100 (Canberra).

Les logiciels mis en oeuvre lors de ces essais sont « Génie 2000 » (Canberra) pour la spectrométrie gamma, ainsi que le logiciel d'analyse MCS (Multi Channel Scaling) « IDSWear » commercialisé par la société Atlantic Nuclear Services (ANS), Canada. Les résultats présentés ci-dessous incluent une correction en demi-vie (qui est 6 heures pour le Tc-99) par le logiciel de mesure « IDSWear ».

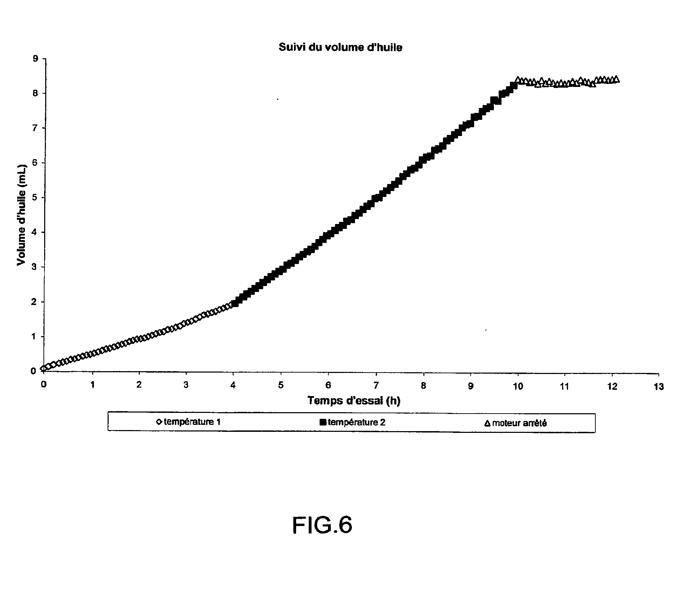

Le diagramme de la figure 6 montre la quantité d'huile piégée dans un dispositif de piégeage situé en aval d'un système de séparation d'huile, en fonction du nombre d'heures d'essais réalisées.

Le volume d'huile piégé a été calculé sur base de l'activité spécifique de l'huile du moteur (qui était de 2 Bq par microlitre en début d'essai), en tenant compte de l'efficacité du détecteur et de la géométrie de détection.

L'efficacité globale sur le pic de 141 keV (environ 6%) a été évaluée à partir du volume d'huile piégé en fin d'essai.

L'augmentation de l'activité détectée en fonction du temps est ainsi convertible en volume d'huile piégée.

Lors de cet essai, trois phases ont été réalisées : une première phase sur un point de fonctionnement stabilisé pour une régulation de température d'huile 1 durant 4 heures, une seconde phase pour une température d'huile 2 durant 6 heures et enfin un contrôle moteur arrêté durant 2 heures. On constate bien trois pentes différentes suivant les trois phases : 37,8 microlitres entre deux points de mesure lors de la première phase, 90,1 microlitres entre deux points de mesure lors de la deuxième phase et 2,2 microlitres entre deux points de mesure lors de la dernière phase.

Pour la dernière phase, la légère augmentation s'explique essentiellement par le léger ruissellement dans le dispositif de piégeage.

## Revendications

1. Procédé de détermination de la consommation d'huile issue du système de séparation d'huile (2) situé dans le circuit de recyclage des gaz de carter d'un moteur à combustion interne, lesdits gaz de carter résultant de la fuite des gaz de combustion hors de la chambre de combustion **caractérisé par le fait que**
• l'on marque l'huile de lubrification dudit moteur par introduction d'au moins un traceur radioactif au sein de ladite huile,
• que l'on fait transiter les gaz de carter, sortant du bloc moteur (1) et chargés d'huile de lubrification, par un système de séparation d'huile (2) où au moins une partie de l'huile contenue au sein desdits gaz est séparée, recueillie et renvoyée vers le carter d'huile (6),
• que l'on piège l'huile non séparée des gaz de carter sortant du système de séparation d'huile (2) dans un dispositif de piégeage d'huile (4) situé en aval dudit système de séparation d'huile (2),
• que l'on mesure, à l'aide d'un détecteur (3), placé à proximité du dispositif de piégeage d'huile (4) et sensible au rayonnement ionisant émis par le ou les traceur(s) radioactif(s), la radioactivité de l'huile non séparée dans le système de séparation (2) d'huile et retenue dans le dispositif de piégeage d'huile (4) ledit détecteur est protégé par un matériau approprié contre le rayonnement émis par l'huile de lubrification en circulation dans le moteur situé à proximité, do manière à ce que ce détecteur (3) mesure uniquement la radioactivité qui s'accumule dans le dispositif de piégeage (4), et
• que l'on transmet les résultats de ces mesures vers un ordinateur (5) apte à calculer à partir de ces résultats la consommation d'huile lubrifiante non séparée dans ledit système de séparation (2).

2. Procédé selon la revendication 1, **caractérisé par le fait que** le système de séparation d'huile (2) est constitué de plusieurs séparateurs, montés en série ou en parallèle.

3. Procédé selon l'une quelconque des revendications précédentes, **caractérisé par le fait que** les gaz de carter sortant du dispositif de piégeage (4) sont libérés dans l'atmosphère ou renvoyés vers l'admission (D) du moteur.

4. Procédé selon l'une quelconque des revendications précédentes, **caractérisé par le fait que** le dispositif de piégeage d'huile (4) est un deuxième système de séparation comportant un ou plusieurs éléments statiques de séparation et/ou un ou plusieurs cyclones et/ou un ou plusieurs éléments filtrants.

5. Procédé selon l'une quelconque des revendications précédentes, **caractérisé par le fait que** le dispositif de piégeage d'huile (4) est conçu de manière à ce que la différence de pression (ΔP) entre l'entrée du système de séparation d'huile (2) et la sortie du système de séparation d'huile (2) est sensiblement identique à la valeur de cette différence de pression en l'absence du dispositif de piégeage.

6. Procédé selon l'une quelconque des revendications précédentes, **caractérisé par le fait que** le traceur radioactif est un composé organique ou minéral d'un élément radioactif.

7. Procédé selon l'une des revendications précédentes, **caractérisé par le fait que** l'élément radioactif a une période, ou demi-vie, inférieure à 3 ans, de préférence inférieure à 1 an, et en particulier inférieure à 30 jours.

8. Procédé selon la revendication 7, **caractérisé par le fait que** l'élément radioactif est choisi parmi le ²²Na, le ⁶⁵Zn, le ⁴⁵Ca, le ³⁵S, le ³²P, le ⁴⁷Ca, le ⁹⁹Mo, le ⁸²Br, le ⁶⁴Cu, le ⁹⁹mTc, le ²⁸Mg, le ⁶⁸Ge, le ⁶⁹Ge, le ⁷⁷Ge, le ⁸⁵Sr et le ⁵⁶Co.

9. Procédé selon la revendication 8, **caractérisé par le fait que** le radiotraceur est choisi parmi les tétra-alkylgermanes contenant du ⁶⁹Ge, de préférence parmi le tétrahexylgermane, le tétraheptylgermane et le tétraoctylgermane, ou un mélange de ceux-ci.

10. Procédé selon la revendication 8, **caractérisé par le fait que** le radiotraceur est le ^{99m}Tc, de préférence sous forme d'une solution aqueuse de pertechnétate de sodium NaTcO₄ ou sous forme de particules de dimensions nanométriques et isolées de l'atmosphère par du carbone.

11. Procédé selon l'une quelconque des revendications précédentes, **caractérisé par le fait que** le détecteur est une sonde de détection de rayonnements ionisants.

12. Dispositif de détermination de la consommation d'huile issue du système de séparation d'huile (2) situé dans le circuit de recyclage des gaz de carter d'un moteur à combustion interne, lesdits gaz de carter résultant de la fuite des gaz de combustion hors de la chambre de combustion, **caractérisé par le fait qu'**il comprend,
• un moteur à combustion interne lubrifié par une huile marquée par introduction d'au moins un traceur radioactif au sein de ladite huile,
• un système de séparation d'huile (2) recevant les gaz de carter chargés en huile de lubrification sortant du bloc moteur (1), où au moins une partie de l'huile contenue dans lesdits gaz de carter est séparée, recueillie et renvoyée vers le carter d'huile,
• en aval du système de séparation d'huile (2), un dispositif de piégeage (4) d'huile,
• un détecteur (3) sensible au rayonnement ionisant émis par le ou les traceur(s) radioactif(s), situé à proximité directe du dispositif de piégeage (4), de manière à permettre la mesure de la radioactivité de l'huile non séparée dans le système de séparation (2) d'huile et retenue dans le dispositif de piégeage d'huile (4) ledit détecteur est protégé par un matériau approprié contre le rayonnement émis par l'huile de lubrification en circulation dans le moteur situé à proximité, de manière à ce que ce détecteur (3) mesure uniquement la radioactivité qui s'accumule dans le dispositif de piégeage (4), et
• relié audit détecteur (3), un ordinateur (5) programmé pour calculer à partir des résultats des mesures de radioactivité, la consommation d'huile lubrifiante non séparée dans ledit système de séparation (2).

13. Dispositif selon la revendication 12, **caractérisé par le fait que** le dispositif de piégeage d'huile (4) est conçu de manière à ce que la différence de pression (ΔP) entre l'entrée et la sortie du système de séparation d'huile (2) est sensiblement identique à la valeur de cette différence de pression en l'absence dudit dispositif de piégeage d'huile.

## Claims

1. Method of determining the consumption of oil coming from the oil separation system (2) located in the circuit for recycling the blow-by gases of an internal combustion engine, said blow-by gases resulting from the leakage of combustion gases out of the combustion chamber, **characterized in that**:
• the lubricating oil for said engine is labelled by introducing at least one radioactive tracer into said oil;
• the blow-by gases, leaving the engine block (1) and laden with lubricating oil, are made to pass through an oil separation system (2) where at least some of the oil contained within said gases is separated, collected and returned to the oil sump (6);
• the oil not separated from the blow-by gases coming from the oil separated system (2) is trapped in an oil trapping device (4) located downstream of said oil separated system (2);
• the radioactivity of the oil not separated in the oil separated system (2) and retained in the oil-trapping device (4) is measured using a detector (3), which is placed near the oil-trapping device (4) and is sensitive to the ionizing radiation emitted by the radioactive tracer (s), said detector is protected by a suitable material from the radiation emitted by the lubricating oil, which is circulating in the engine located nearly, so that this detector (3) measures only the radioactivity that accumulates in the trapping devices (4); and
• the results of these measurements are sent to a computer (5) capable of calculating the consumption of lubricating oil not separated in said separated system (2) from these results.

2. Method according to claim 1, **characterized in that** the oil separation system (2) consists of several separators connected in series or in parallel.

3. Method according to either of the preceding claims, **characterized in that** the blow-by gases coming from the trapping device (4) are released into the atmosphere or sent to the intake (D) of the engine.

4. Method according to any one of the preceding claims, **characterized in that** the oil-trapping device (4) is a second separation system comprising one or more static separation elements and/or one or more cyclones and/or one or more filtering elements.

5. Method according to any one of the preceding claims, **characterized in that** the oil-trapping devise (4) is designed so that the pressure difference (ΔP) between the inlet of the oil separation system (2) and the outlet of the oil separation system (2) is substantially the same as the value of this pressure difference in the absence of the trapping device.

6. Method according to any one of the preceding claims, **characterized in that** the radioactive tracer is an organic or mineral compound of a radioactive element.

7. Method according to one of the preceding claims, **characterized in that** the radioactive element has a period, or half-life, of less than 3 years, preferably less than 1 year and in particular less than 30 days.

8. Method according to Claim 7, **characterized in that** the radioactive element is chosen from ²²Na, ⁶⁵Zn, ⁴⁵Ca, ³⁵S, ³²P, ⁴⁷Ca, ⁹⁹Mo, ⁸²Br, ⁶⁴Cu, ^{99m}Tc, ²⁸Mg, ⁶⁸Ge, ⁶⁹Ge, ⁷⁷Ge, ⁸⁵Sr, ⁵⁶Co

9. Method according to Claim 8, **characterized in that** the radiotracer is chosen from tetra-alkylgermanes containing ⁶⁹Ge, preferably from tetrahexylgermane, tetraheptylgermane and tetraoctylgermane, or a mixture thereof.

10. Method according to Claim 8, **characterized in that** the radiotracer is ^{99m}Tc, preferably in the form of an aqueous solution of sodium pertechnetate NaTcO₄ or in the form of nanoscale particles isolated from the atmosphere by carbon.

11. Method according to any one of the preceding claims, **characterized in that** the detector is an ionizing radiation detection probe.

12. Device for determining the consumption of oil coming from the oil separation system (2) located in the circuit for recycling the blow-by gases of an internal combustion engine, said blow-by gases resulting from the leakage of combustion gases out of the combustion chamber, **characterized in that** it comprises:
• an internal combustion engine lubricated by an oil labelled by introducing at least one radioactive tracer into said oil;
• an oil separation system (2) that receives the blow-by gases laden with lubricating oil leaving the engine block (1), where at least some of the oil contained in said blow-by gases is separated, collected and returned to the oil sump;
• downstream of the oil separation system (2), an oil-trapping device (4);
• a detector (3) sensitive to the ionizing radiation emitted by the radioactive tracer(s), located in the immediate vicinity of the trapping device (4), so as to measure the radioactivity of the oil not separated in the oil separation system (2) but retained in the oil-trapping device (4), said detector is protected by a suitable material from the radiation emitted by the lubricating oil, which is circulating in the engine located nearby, so that this detector (3) measures only the radioactivity that accumulates in the trapping device (4); and
• connected to said detector (3), a computer (5) programmed for calculating the consumption of lubricating oil not separated in said separation system (2) from the results of the radioactivity measurements.

13. Device according to Claim 12, **characterized in that** the oil-trapping device (4) is designed in such a way that the pressure difference (ΔP) between the inlet and the outlet of the oil separation system (2) is approximately the same as the value of this pressure difference in the absence of said oil-trapping device.

## Patentansprüche

1. Verfahren zur Bestimmung des Ölverbrauchs, der von einem in dem Kreislauf zum Recycling der Gehäusegase eines internen Verbrennungsmotors befindlichen System zur Ölabtrennung (2) herrührt, wobei die Gehäusegase aus dem Entweichen der Verbrennungsgase aus dem Brennraum resultieren, **dadurch gekennzeichnet, dass**
- das Schmieröl des Motors durch Einführen von mindestens einem radioaktiven Tracer in das Öl markiert wird,
- die den Motorblock (1) verlassenden und mit Schmieröl beladenen Gehäusegase durch ein System zur Ölabtrennung (2) befördert werden, wo mindestens ein Teil des in den Gasen enthaltenen Öls abgetrennt, gesammelt und zum Ölsumpf (6) zurückgeführt wird,
- das Öl, das nicht von den aus dem System zur Ölabtrennung (2) austretenden Gehäusegasen abgetrennt wird, in einer stromabwärts des Systems zur Ölabtrennung (2) befindlichen Ölabscheidevorrichtung (4) abgeschieden wird,
- mit Hilfe einer Detektors (3), der in der Nähe der Ölabscheidevorrichtung (4) angeordnet wird und empfindlich gegenüber der durch den oder die radioaktiven Tracer emittieren ionisierenden Strahlung ist, die Radioaktivität des nicht in dem System zur Ölabtrennung (2) abgetrennten und in der Ölabscheidevorrichtung (4) zurückbehaltenen Öls gemessen wird, wobei der Detektor mit einem Material geschützt wird, das gegenüber der Strahlung, die durch das im in der Nähe angeordneten Motor umlaufende Schmieröl emittiert wird, so angepasst wird, dass der Detektor (3) ausschließlich die Radioaktivität misst, die sich in der Abscheidevorrichtung (4) akkumuliert, und
- die Ergebnisse dieser Messungen an einen Computer (5) übermittelt werden, der in der Lage ist, ausgehend von diesen Ergebnissen den Verbrauch an nicht in dem System zur Abtrennung (2) abgetrennten Schmieröl zu berechnen.

2. Verfahren gemäß Anspruch 1, **dadurch gekennzeichnet, dass** das System zur Ölabtrennung (2) aus mehreren Abtrennen besteht, die in Serie oder parallel montiert sind.

3. Verfahren gemäß irgendeinem der vorangehenden Ansprüche, **dadurch gekennzeichnet, dass** die die Abscheidevorrichtung (4) verlassenden Gehäusegase in die Atmosphäre freigesetzt werden oder zum Einlass (D) des Motors befördert werden.

4. Verfahren gemäß irgendeinem der vorangehenden Ansprüche, **dadurch gekennzeichnet, dass** die Ölabscheidevorrichtung (4) ein zweites Abtrennsystem ist, das ein oder mehrere statische Abtrennelement (e) und/oder ein oder mehrere Filterelement (e) umfasst.

5. Verfahren gemäß irgendeinem der vorangehenden Ansprüche, **dadurch gekennzeichnet, dass** die Ölabscheidevorrichtung (4) so ausgebildet wird, dass die Druckdifferenz (ΔP) zwischen dem Einlass des Systems zur Ölabtrennung (2) und dem Auslass des Systems zur Ölabtrennung (2) im Wesentlichen identisch ist mit dem Wert dieser Druckdifferenz bei Abwesenheit der Abscheidevorrichtung.

6. Verfahren gemäß irgendeinem der vorangehenden Ansprüche, **dadurch gekennzeichnet, dass** der radioaktive Tracer eine organische oder mineralische Verbindung eines radioaktiven Elements ist.

7. Verfahren gemäß irgendeinem der vorangehenden Ansprüche, **dadurch gekennzeichnet, dass** das radioaktive Element eine Zeitdauer oder Halbwertszeit von weniger als drei Jahren, bevorzugt weniger als ein Jahr und insbesondere weniger als 30 Tage aufweist.

8. Verfahren gemäß Anspruch 7, **dadurch gekennzeichnet, dass** das radioaktive Element aus ²²Na, ⁶⁶Zn, ⁴⁵Ca, ³⁵S, ³²P, ⁴⁷Ca, ⁹⁹Mo, ⁸²Br, ⁶⁴Cu, ^{99m}Tc, ²⁸Mg, ⁶⁸Ge, ⁶⁹Ge, ⁷⁷Ge, ⁸⁵Sr und ⁵⁶Co ausgewählt wird.

9. Verfahren gemäß Anspruch 8, **dadurch gekennzeichnet** das der Radiotracer aus den ⁶⁹Ge enthaltenden Tetraalkylgermanen, bevorzugt aus den Tetrahexylgermanen, den Tetraheptylgermanen und den Tetraoktylgermanen, oder einer Mischung davon ausgewählt wird.

10. Verfahren gemäß Anspruch 8, **dadurch gekennzeichnet, dass** der Radiotracer ^{99m}Tc ist, bevorzugt in Form einer wässrigen Lösung von Natriumpertechnat NaTcO₄ oder in Form von Partikeln mit manometrischen Abmessungen, die mit Kohlenstoff aus der Atmosphäre isoliert werden.

11. Verfahren gemäß irgendeinem der vorangehenden Ansprüche, **dadurch gekennzeichnet, dass** der Detektor eine Sonde zur Bestimmung von ionisierenden Strahlen ist.

12. Vorrichtung zur Bestimmung des Ölverbrauchs, der von einem im Kreislauf zum Recycling der Gebäusegase eines internen Verbrennungsmotors befindlichen System zur Ölabtrennung (2) herrührt, wobei die Gehäusegase vom Entweichen der Verbrennungsgase aus dem Brennraum resultieren, **dadurch gekennzeichnet, dass** sie umfasst :
- einen internen Verbrennungsmotor, der mit einem durch Einführen von mindestens einem radioaktiven Tracer in das Öl markierten Öl geschmiert ist,
- ein die den Motorblock (1) verlassenden, mit Schmieröl beladenen Gehäusegase aufnehmendes System zum Ölabtrennen (2), worin mindestens ein Teil des in den Gehäusegasen enthaltenen Öls abgetrennt, gesammelt und zum Ölsumpf zurückgeführt wird,
- einen Ölabscheider (4) stromabwärts des Systems zur Ölabtrennung (2),
- einen gegenüber der durch den oder die radioaktiven Tracer emittierten ionisierenden Strahlung empfindlichen
Detektor (3), der sich in direkter Nähe zur Abscheidevorrichtung (4) befindet, so dass die Messung der Radioaktivität des nicht in dem System zur Ölabtrennung (2) abgetrennten und in der Ölabscheidevorrichtung (4) zurückgehaltenen Öl ermöglicht ist, wobei der Detektor durch ein Material geschützt ist, das gegenüber der Strahlung, die durch das im in der Nähe liegenden Motor umlaufende Schmieröl emittiert wird, so angepasst ist, dass der Detektor (3) ausschließlich die Radioaktivität misst, die in der Abscheidevorrichtung (4) akkumuliert ist, und
- ein mit dem Detektor (3) verbundener Computer (5),
der zum Berechnen des Verbrauchs an nicht in dem System zur Abtrennung (2) abgetrennten Schmieröl ausgehend von den Ergehnissen der Radioaktivitätsmessungen programmiert ist.

13. Vorrichtung gemäß Anspruch 12, **dadurch gekennzeichnet, dass** die Ölascheidevorrichtung (4) so ausgebildet ist, dass die Druckdifferenz (ΔP) zwischen dem Einlass und dem Auslass des Systems zur Ölabtrennung (2) ungefähr gleich dem Wert dieser Druckdifferenz bei Abwesenheit der Ölabscheidevorrichtung ist.
